# EUROPEAN PATENT SPECIFICATION

(11) **EP 2 613 817 B1**
(45) Date of publication and mention of the grant of the patent: **02.03.2016**
(21) Application number: 11752046.0
(22) Date of filing: 24.08.2011
(51) Int. Cl.: A61L 27/04, A61L 27/30, A61L 31/02, A61L 31/08, C22C 23/02

(54) **BIOERODIBLE MAGNESIUM ALLOY CONTAINING ENDOPROSTHESES**
BIOLOGISCH MAGNESIUMLEGIERUNG ENTHALTENDE ENDOPROTHESEN
ENDOPROTHÈSES CONTEINT UNE ALLIAGE DE MAGNESIUM BIODÉGRADABLE

(30) Priority: 07.09.2010 US 380435 P
(43) Date of publication of application: 17.07.2013
(73) Proprietor: Boston Scientific Scimed, Inc., Maple Grove, MN 55311-1566 (US)
(72) Inventor: WEBER, Jan, 6228 Maastricht (NL); FLANAGAN, Aiden, County Kilcolgan Galway (IE); SCHEUERMANN, Torsten, 80803 Munich (DE)
(74) Representative: Peterreins Schley
(86) International application number: PCT/US2011/048954
(87) International publication number: WO 2012/033637

(56) References cited:
- WO-A1-2008/034066
- WO-A2-2008/091835
- ERINC M ET AL: "Applicability of existing magnesium alloys as biomedical implant materials", MAGNESIUM TECHNOLOGY 2009, PROCEEDINGS OF THE SYMPOSIUM HELD DURING THE TMS ANNUAL MEETING & EXHIBITION, SAN FRANCISCO, CA, UNITED STATES, FEB. 15-19, 2009,, 15 February 2009 (2009-02-15), pages 209-214, XP009119997, ISBN: 978-0-87339-730-8
- SILLEKENS ET AL: "Modified AZ80 Magnesium Alloys for Biomedical Applications", MAGNESIUM TECHNOLOGY,, 14 February 2010 (2010-02-14), pages 641-646, XP009152937,

## Description

### TECHNICAL FIELD

This disclosure relates to an endoprostheses that includes a bioerodible magnesium alloy.

### BACKGROUND

Endoprostheses can be used to replace a missing biological structure, support a damaged biological structure, and/or enhance an existing biological structure. Frequently, only a temporary presence of the endoprosthesis in the body is necessary to fulfill the medical purpose. Permanent placement of endoprostheses can result in unwanted biological reactions in the long term, even with the most highly biocompatible permanent materials. Surgical intervention to remove endoprostheses, however, can cause complications and may not even be possible. One approach for avoiding permanent presence of all or part of an endoprosthesis is to form all or part of the endoprosthesis out of bioerodible material. The term "bioerodible" as used herein is understood as the sum of microbial procedures or processes solely caused by the presence of endoprosthesis within a body, which results in a gradual erosion of the structure formed of the bioerodible material.

At a specific time, the endoprosthesis, or at least the part of the endoprosthesis which comprises the bioerodible material, loses its mechanical integrity. The erosion products are mainly absorbed by the body, although small residues can remain under certain conditions. A variety of different bioerodible polymers (both natural and synthetic) and bioerodible metals (particularly magnesium and iron) have been developed and are under consideration as candidate materials for particular types of endoprostheses. Many of these bioerodible materials, however, have significant drawbacks. These drawbacks include the erosion products, both in type and in rate of release, as well as the mechanical material properties of the material. Erosion products, if released in a sufficient quantity in a short enough time interval, can result in unwanted biological reactions. ERINC M ET AL, "Applicability of existing magnesium alloys as biomedical implant materials", MAGNESIUM TECHNOLOGY 2009, PROCEEDINGS OF THE SYMPOSIUM HELD DURING THE TMS ANNUAL MEETING & EXHIBITION, SAN FRANCISCO, CA, UNITED STATES, FEB. 15-19, 2009, pages 209-214, discloses modified AZ80 magnesium alloys for biomedical applications.

### SUMMARY

A bioerodible stent is described that includes a bioerodible magnesium alloy as defined in appended claim 1. The stent further has a coating that consists of titanium oxide and can optionally contain an organic compound.

The bioerodible magnesium alloy includes less than 5 weight percent of elements other than magnesium, aluminum, zinc, and manganese. The bioerodible magnesium alloy can also include less than 2 weight percent of elements other than magnesium, aluminum, zinc, and manganese. In other embodiments, the bioerodible magnesium alloy consists essentially of magnesium, aluminum, zinc, and manganese. In some embodiments, the iron content of the bioerodible magnesium alloy is less than 50 ppm Fe.

The bioerodible magnesium alloy can optionally include one or more rare earth metals. In some embodiments, the alloy includes between 0.1 and 1.5 weight percent of a first rare earth metal. The bioerodible magnesium alloy can include between 0.1 and 0.8 weight percent of the first rare earth metal. In some embodiments, the first rare earth metal is selected from the group of yttrium, neodymium, lanthanum, and cerium. In some embodiments, the bioerodible magnesium alloy also includes between 0.1 and 1.5 weight percent of a second rare earth metal. In some embodiments, the bioerodible magnesium alloy includes less than 3 weight percent of elements other than magnesium, aluminum, zinc, manganese, and rare earth metals. The bioerodible magnesium alloy can also consist essentially of magnesium, aluminum, zinc, manganese, and rare earth metals.

The stent includes a coating. In some embodiments, the coating has a maximum thickness of 20 nm. The coating can be essentially free of pores. In some embodiments, the coating includes an organic compound deposited by molecular layer deposition.

The stent can also include a therapeutic agent. The therapeutic agent can be deposited over the coating. In some embodiments, a barrier layer of aluminum oxide can be deposited by atomic layer deposition over the therapeutic agent and the coating.

One advantage of the bioerodible magnesium alloys described herein is that they have a slower bioerosion rate than pure magnesium and many bioerodible magnesium alloys. The slower erosion rate is a result of the enhanced corrosion resistance of the bioerodible magnesium alloy. A slower erosion rate can allow an endoprosthesis to retain its mechanical integrity for a longer period of time for a particular dimension. For example, when used in a stent the enhanced corrosion resistance of the bioerodible magnesium alloy can lengthen the time during which the stent can provide sufficient scaffolding ability *in vivo.* The enhanced corrosion resistance also reduces the amount of hydrogen evolution.

Another advantage of the bioerodible magnesium alloys described herein is that they, and their erosion products, are biocompatible.

Another advantage of the bioerodible magnesium alloys described herein is that they have a desirable combination of mechanical properties for use in endoprostheses, particularly stents, such as strength, ductility and strain hardening. The bioerodible magnesium alloy also has good processability. For example, a stent produced out of the bioerodible magnesium alloy will have good tube drawing properties during production and good scaffolding strength in the final stent.

In some aspects, the stent allows endothelialization to occur before the bioerodible magnesium alloy begins to erode significantly. Endothelialization can help prevent debris from the eroding stent from being transported through the blood stream. Endothelialization can also block the oxygen-rich turbulent flow of the blood stream from contacting the stent, thus further reducing the erosion rate of the stent.

The details of one or more embodiments are set forth in the accompanying drawings and the description below. Other features, objects, and advantages will be apparent from the description and drawings, and from the claims.

### DESCRIPTION OF DRAWINGS

FIG. 1 is a perspective view of a representative stent.
FIGS. 2A and 2B depict the stress/strain curve for certain magnesium alloys.
FIG. 3 depicts the corrosion rates for certain magnesium alloys.
FIGS. 4A and 4B depict explants of peritoneal implanted bare magnesium rods in mice after 30 days.
FIGS. 5A-5D show examples of cross-sections of stent struts according to different embodiments.

### DETAILED DESCRIPTION

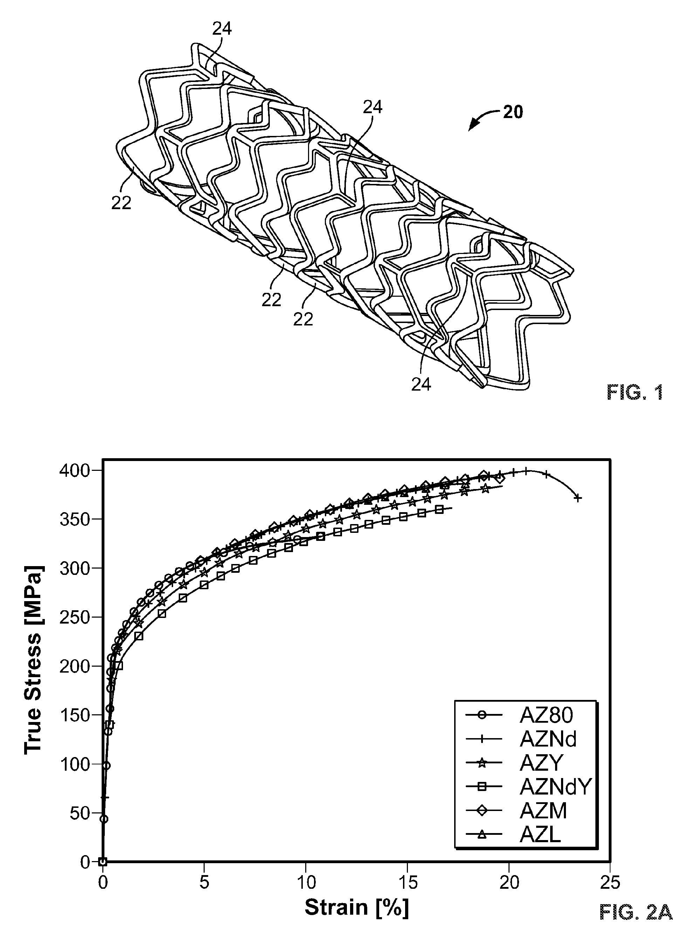

A stent 20, shown in Fig. 1, is discussed below as an example stent according to the instant disclosure. Stent 20 includes a pattern of interconnected struts forming a structure that contacts a body lumen wall to maintain the patency of the body lumen. For example, stent 20 can have the form of a tubular member defined by a plurality of bands 22 and a plurality of connectors 24 that extend between and connect adjacent bands. During use, bands 22 can be expanded from an initial, small diameter to a larger diameter to contact stent 20 against a wall of a vessel, thereby maintaining the patency of the vessel. Connectors 24 can provide stent 20 with flexibility and conformability that allow the stent to adapt to the contours of the vessel. Other examples of endoprostheses can include covered stents and stent-grafts.

One or more struts of stent 20 is adapted to erode under physiological conditions. Accordingly, stent 20 includes the bioerodible magnesium alloy of the invention. The bioerodible magnesium alloy includes less than 5 weight percent, in sum, of elements other than magnesium, aluminum, zinc, and manganese. In some embodiments, the bioerodible magnesium alloy includes less than 2 weight percent, in sum, of elements other than magnesium, aluminum, zinc, and manganese.

The bioerodible magnesium alloy can consist essentially of magnesium, aluminum, zinc, and manganese. As used herein, "consisting essentially of" means that the alloy can also include impurities normally associated with the commercially available forms of the constituent elements in amounts corresponding to the amounts found in the commercially available forms of the constituent elements. In some embodiments, the potential impurity elements of iron, copper, nickel, gold, cadmium, bismuth, sulfur, phosphorous, silicon, calcium, tin, lead and sodium are each maintained at levels of less than 1000 ppm. In still other embodiments, the potential impurity elements of iron, copper, nickel, cobalt, gold, cadmium, bismuth, sulfur, phosphorous, silicon, calcium, tin, lead and sodium are each maintained at levels of less than 200 ppm. Iron, nickel, copper, and cobalt have low solid-solubility limits in magnesium and can serve as active cathodic sites and accelerate the erosion rate of magnesium within a physiological environment. In still other embodiments, each of iron, nickel, copper, and cobalt is maintained at levels of less than 50 ppm. For example, each of the first five alloys listed in Table I has no more than 35 ppm of iron.

In some embodiments, the bioerodible magnesium alloy includes between 0.1 and 1.5 weight percent of a first rare earth metal. In other embodiments, the alloy includes between 0.1 and 0.8 weight percent of the first rare earth metal. In some embodiments, the first rare earth metal is yttrium, neodymium, lanthanum, or cerium. Rare earth metals can increase the ductility and improve the strain refining of the alloy, which can improve the processability of the alloy. The bioerodible magnesium alloy can also include between 0.1 and 1.5 weight percent of a second rare earth metal. For example, the bioerodible magnesium alloy can include about 0.5 weight percent yttrium and 0.6 weight percent neodymium. In some embodiments, the bioerodible magnesium alloy includes three or more rare earth metals.

In still other embodiments, the bioerodible magnesium alloy includes no more than 0.8 weight percent of any rare earth metal. In some embodiments, the total amount of rare earth metals within the bioerodible magnesium alloy is maintained at a level of less than 10.0 weight percent. In some embodiments, the total amount of rare earth metals within the bioerodible magnesium alloy is maintained at a level of less than 2.5 weight percent. The addition of the rare earth metals in amounts of between 0.1 and 0.8 weight percent will not significantly increase the corrosion rate of the bioerodible magnesium alloy, but can improve the mechanical properties of the alloy.

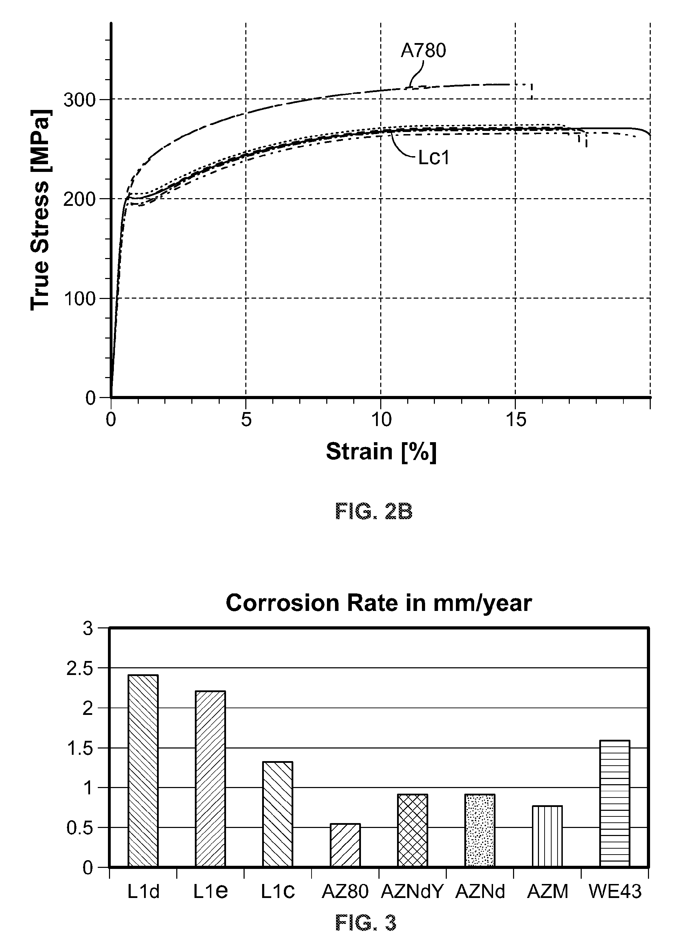

Specific examples of bioerodible magnesium alloys are shown in Table I. Table II includes a number of magnesium alloys for comparative purposes. Table III shows the average corrosion potential, current density, and corrosion rates for each of the alloys from Table I. FIG. 2A depicts the stress/strain curve for each of these elements of Table I. FIG. 2B depicts the stress/strain curve of AZ80 verses Lc1. FIG. 3 depicts the corrosion rates for some of the magnesium alloys of Tables I and II. As can be seen, the AZ80, AZNdY, AZNd, and AZM alloys all have an erosion rate that is lower than the erosion rates of L1d, L1e, L1c, and WE43. The erosion rate of AZ80 is less than half the erosion rate of WE43.

**Table I**

| Alloy | Al | Zn | Mn | Y | Nd | La | Fe | Mg |
|---|---|---|---|---|---|---|---|---|
| AZ80 | 7.5 | 0.5 | 0.2 | -- | -- | -- | 35 ppm | Balance |
| AZNd | 7.3 | 0.6 | 0.1 | -- | 0.7 | -- | 35 ppm | Balance |
| AZY | 7.4 | 0.6 | 0.1 | 0.5 | -- | -- | 35 ppm | Balance |
| AZNdY | 7.0 | 0.6 | 0.2 | 0.5 | 0.6 | -- | 30 ppm | Balance |
| AZM | 7.3 | 0.6 | 0.4 | -- | -- | -- | 32 ppm | Balance |
| AZL | 7.0 | 0.5 | 0.2 | -- | -- | 1.2 | 53 ppm | Balance |

**Table II**

| Alloy | Zn | Zr | Mn | Y | Nd | Ca | Ag | Fe | Other Elements | Mg |
|---|---|---|---|---|---|---|---|---|---|---|
| L1c | 2.87 | <0.02 | 0.15 | -- | -- | 0.22 | 0.10 | 0.0036 | -- | Balance |
| L1d | 2.86 | 0.28 | 0.14 | -- | -- | 0.21 | 0.10 | 0.0022 | -- | Balance |
| L1e | 2.88 | <0.02 | 0.15 | -- | -- | 0.11 | 0.10 | 0.0036 | -- | Balance |
| WE43 | Not specified | 0.0-1.0 | Not specified | 2.0 - 6.0 | 1.5 - 4.5 | Not specified | Not specified | Not specified | 0.5 - 4.0 of other rare earths metals; 0.0-03 Al | Balance |

**Table III**

| Alloy | E_{corr} [V/Ag-AgCl] | I_{corr} [µA/cm²] | Average Corrosion Rate [mm/y] |
|---|---|---|---|
| AZ80 | -1.49 ± 0.01 | 29.5 ± 5.1 | 0.65 ± 0.11 |
| AZNd | -1.61 ± 0.14 | 41.4 ± 4.5 | 0.91 ± 0.10 |
| AZY | -1.48 ± 0.01 | 41.9 ± 3.9 | 0.93 ± 0.090.140.15 |
| AZNdY | -1.57 ± 0.02 | 41.5 ± 6.2 | 0.91 ± 0.13 |
| AZM | -1.51 ± 0.02 | 35.0 ± 2.2 | 0.77 ± 0.05 |
| AZL | -1.59 ± 0.01 | 43.0 ± 4.4 | 0.95 ± 0.09 |

FIGS. 2A and 2B depict the stress/strain curve for AZ80, Lc1, ALZ, and the other alloys in Table I after drawing the alloy into a vascotube. These stress/strain curves show that the alloys of Table I have suitable mechanical properties for being used in a stent. In particular, each alloy has an upper yield point at a stress of somewhere between about 200 and 250 MPa and an ultimate tensile point at a stress of somewhere between about 300 and 400 MPa after a strain of between 10 and 25 percent. Stents can be significantly deformed during crimping and expansion, thus strains of 10 to 25 percent, depending on the particular stent design, are possible. Materials permitting more strain can be used in stent designs having sharper corners, which can be crimped to smaller dimensions than stents having more gradual corners. Moreover, a material having a ultimate tensile point at a stress greater than the stress for the upper yield point permits for the stent to be uniformly expanded while minimizing the risk that some parts of the stent will be deformed until they break while other parts of the stent remain unexpanded. Furthermore, the upper yield point of the materials of Table I at between about 200 and 250 MPa are sufficiently high to maintain a radial force to keep the vessel open, and thus avoid plastic deformation of the stent once implanted. Additionally, the initial slope of the stress/strain curve for each material of Table I is sufficiently steep to minimize the relaxation of the stent back to a smaller diameter immediately after expansion at the implantation site. Relaxation of the stent immediately after expansion, if too great, can leave a gap between stent and vessel wall.

The stress/strain curve of FIG. 2A also shows that the alloys including rare earth metals have a longer elongation at break than AZ80, which indicates a greater ductility. AZ80 has a high ultimate tensile strength to yield strength ratio, which creates a stent that can be fully expanded. If the ultimate tensile strength is approximately the same as the yield strength, the stent may fracture before the stent is fully expanded. L1c and AZ80 have comparable ultimate strain rates.

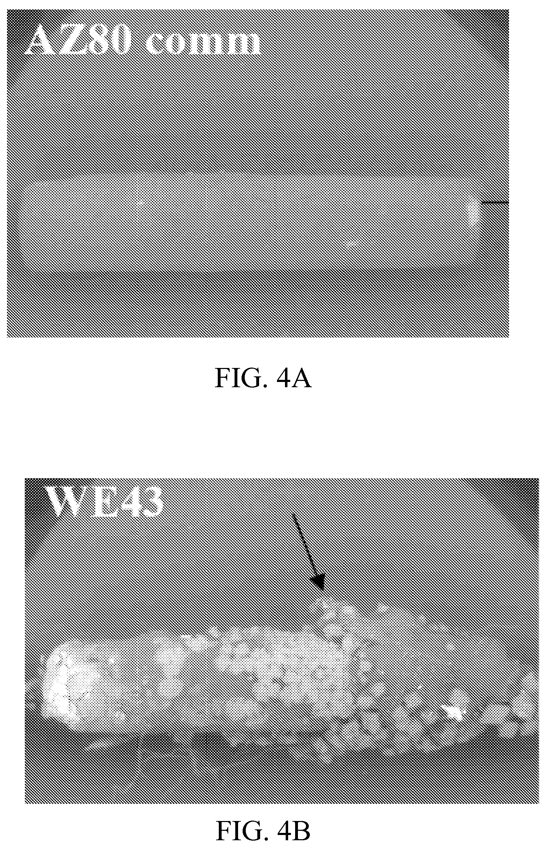

FIGS. 4A and 4B depict explants of peritoneal implanted bare magnesium rods in mice after 30 days. FIG. 4A depicts a rod of the AZ80 alloy, while FIG. 4B depicts a rod of the WE43 alloy. In the experiment, 4 mm long rods with a 1 mm diameter were implanted subcutaneous and interperitonial in mice to study the in-vivo biodegradation. Rods, as compared to stents, require fewer shaping steps and the interperitonial implantation of a rod can mimic a vascular fluid environment. After 30 days in a mouse, the AZ80 rod had a magnesium hydroxide coating. After 30 days in a mouse, the faster corroding WE43 rod is covered by magnesium phosphate crystals. The magnesium phosphate crystals may be due to an local overload of Mg ions which reacts with the phosphate ions in the surrounding fluid. In case of the slower corroding AZ80, the rate of magnesium ions being produced may be sufficiently low that magnesium ions can be removed systemically without forming local crystals. Magnesium phosphate within a vascular environment may be replaced by calcium deposits, and thus may cause calcifications at the implant site. Accordingly, the AZ80 alloy appears to minimize the adverse reaction associated with having a bioerodible magnesium alloy implanted within a body when compared to faster eroding magnesium alloys, such as WE43.

A coating can be applied to slow or delay the initial degradation of the bioerodible magnesium alloy upon placement within a physiological environment. Delaying the bioerosion processes can allow the body passageway to heal and the stent to become endothelialized (surrounded by tissues cells of the lumen wall) before the strength of the stent is reduced to a point where the stent fails under the loads associated with residing within a body lumen (e.g., within a blood vessel). When an endothelialized stent fragments, the segments of the stent can be contained by the lumen wall tissue and are thus less likely to be released into the blood stream. Endothelialization can also block the oxygen-rich turbulent flow of the blood stream from contacting the endoprosthesis, thus further reducing the erosion rate of the endoprosthesis.

The coating can include one or more layers. In some embodiments, the coating is continuous and essentially non-porous. The coating can be formed by a self-limiting deposition process. In a self-limiting deposition process, the growth of the coating monolayer stops after a certain point (e.g., because of thermodynamic conditions or the bonding nature of the molecules involved), even though sufficient quantities of deposition materials are still available. For example, US Provisional Patent Application 61/228,264, entitled "Medical Devices Having an Inorganic Coating Layer Formed by Atomic Layer Deposition," filed July 24, 2009, describes a process of atomic layer deposition (also known as atomic layer epitaxy). Molecular layer deposition processes can also be used, for example, when depositing an organic layer. Other methods include pulsed plasma-enhanced chemical vapor deposition (*see* Seman et al., Applied Physics Letters 90:131504 (2007)) and irradiation-induced vapor deposition.

By using a self-limiting deposition process to form the coating, such as atomic layer deposition, the coating can have more uniformity in thickness across different regions of the bioerodible magnesium stent and/or a higher degree of conformality. A conformal coating is possible even for surfaces having very high aspect ratio structures (such as deep and narrow trenches or nanoparticles). As used herein, "conformal" means that the coating follows the contours of the medical device geometry and continuously covers over substantially all the surfaces of the medical device.

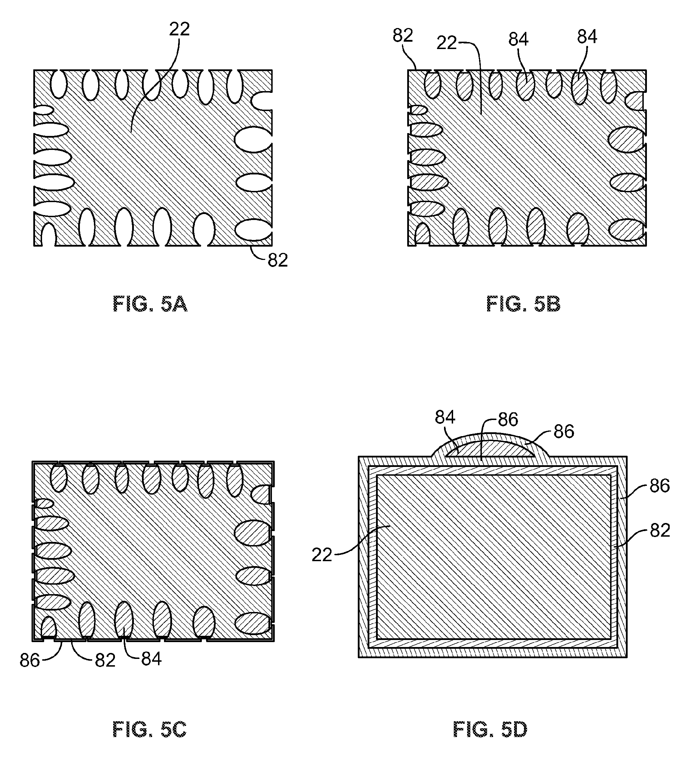

For example, FIG. 5A depicts a cross-section of a stent strut 22 with a highly structured porous surface and a conformal coating 82 that coats the inner surfaces of the pores and fully protects the bioerodible magnesium alloy of the stent strut. In other embodiments, only the abluminal surface of the stent strut is porous and the remaining sides of the strut are smooth. The porous structure can be produced by pneumatically projecting nanoparticles onto a surface of the bioerodible magnesium alloy. For example, the nanoparticles can be charge magnesium nanoparticles. Other coating methods would present a coating challenge for such a structure. With line-of-sight coating processes (e.g., spray coating), there may be a gap in coverage or disproportionately thin coatings. Alternatively, in liquid phase processes such as dip coating or sol-gel, the coating fluid may accumulate in the pores due to surface tension.

The coating consists of titanium oxide and can optionally contain an organic compound. Titanium oxide coatings are biocompatible and have low thrombogenicity. As compared to a bare metal stainless steel stent, a titanium oxide coated stent can achieve about twice the endothelial cell surface coverage after a period of about seven days. Moreover, as compared to a poly(b-styrene-b-isobutylene-b-styrene) (SIBS) coated stent, a titanium oxide coated stent can achieve about four times the endothelial cell coverage after a period of about seven days. Moreover, titanium oxide can also form a strong bond to an underlying magnesium alloy surface and is resistant to erosion away from the surface.

A coating of titanium oxide can be formed by atomic layer deposition, for example, by using titanium tetrachloride (TiCl₄) and water (H₂O) as the precursor materials for producing a titanium oxide coating. For example, the process could involve the following two sequential half-reactions:
(A) :Mg-OH + TiCl₄ (g) → :Mg-O-TiCl₃ + HCl
(B) :Mg-O-TiCl₃ + 3 H₂O → :Mg-O-Ti(OH)₃ + 3 HCl
with :Mg-OH and :Mg-O-TiCl₃ being the surface species. These two half-reactions give the overall reaction :Mg-OH + TiCl₄ + 3 H₂O → :Mg-O-Ti(OH)₃ + 4 HCl. Titanium tetrachloride and other precursor materials for forming a titanium oxide coating can be obtained from Sigma-Aldrich Corporation of St. Louis, MO.

The biocompatibility, porosity, surface interface, and/or corrosion resistance of titanium oxide coatings can also depend upon its crystal structure. In this regard, titanium oxide may exist in an amorphous or crystalline form. In atomic layer deposition, the crystalline anatase form of titanium oxide preferentially develops at relatively higher deposition temperatures (e.g., greater than 250 °C), whereas the amorphous form of titanium oxide preferentially develops at relatively lower deposition temperatures (e.g., less than 150 °C).

The coating can also include a multilayered structure. The multilayered structure can be made of alternating layers of different materials. For example, a coating can include alternating layers of aluminum oxide and titanium oxide. In some embodiments, a multilayered structure can have different levels of porosity and/or crystalline structure. For example, the multilayered structure could include multiple layers of titanium oxide with some layers being amorphous and non-porous, and other layers being crystalline and porous. By controlling the deposition of each monolayer to control the porosity, a desired initial erosion delay and/or reduction can be tailored for a particular use.

The coating can include organic materials. Molecular layer deposition can be used to deposit coatings of organic materials, including 3-(aminopropyl) trimethoxysiloxane and polyimides, such as 1,2,3,5-benzenetetracarboxylic anhydride-4,4-oxydianiline (PMDA-ODA) and 1,2,3,5-benzenetetracarboxylic anhydride-1,6-diaminohexane (PMDA-DAH). In molecular layer deposition, monomers react with an exposed substrate surface in a self-limiting reaction that also results in monolayers. Molecular layer deposition can also be controlled to result in specific porosities. In some embodiments, the coating can include a composite of an atomic layer deposition deposited inorganic material (such as aluminum oxide or titanium oxide) and one or more molecular layer deposition deposited polymers.

Coating medical devices by atomic layer deposition or molecular layer deposition can also permit batch processing to improve manufacturing efficiency and/or process reliability. Multiple stents can be placed into a coating chamber to simultaneously coat the stents by atomic layer deposition or molecular layer deposition. Also, because this may allow multiple stents to be subjected to the same deposition conditions, process reliability can be improved because substantially the same coating can be applied to each stent.

An inorganic coating in accordance with the present disclosure may have various thicknesses, depending upon the particular application. If the coating is too thick, it may crack upon expansion and implantation of the stent. According, in some embodiments, the thickness of the inorganic coating is less than 30 nm, and in some cases, less than 20 nm. The inorganic coating may be as thin as 0.5 nm, but other thicknesses are also possible. More particularly, the coating can be between 1 and 10 nm thick. In still further embodiments, the coating is about 5 nm thick.

The coating can also include a variable thickness. For example, during the atomic layer deposition process, portions of the stent can be selectively masked and/or portions of a coating removed between alternating steps of depositing mono-layers. Areas of different thicknesses can help ensure that physiological fluids do make contact with the underlying bioerodible magnesium alloy after the desired erosion delay period. Moreover, thinner coating portions can allow for the coating to fracture along predetermined lines. For example, portions of a non-porous titanium oxide coating may remain in the body long after the bioerodible magnesium alloy has eroded away, thus it may be beneficial to ensure that the coating breaks up to small and regular sized pieces. In some embodiments, alternating mono-layers can be porous and non-porous and the thinner portions of the coating can be porous to help ensure exposure of the bioerodible magnesium alloy to biological fluids.

The stent can optionally include a therapeutic agent. The therapeutic agent used in the present invention may be any pharmaceutically acceptable agent (such as a drug), a biomolecule, a small molecule, or cells. Exemplary drugs include anti-proliferative agents such as paclitaxel, sirolimus (rapamycin), tacrolimus, everolimus, biolimus, and zotarolimus. Exemplary biomolecules include peptides, polypeptides and proteins; antibodies; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Exemplary small molecules include hormones, nucleotides, amino acids, sugars, lipids, and compounds have a molecular weight of less than 100kD. Exemplary cells include stem cells, progenitor cells, endothelial cells, adult cardiomyocytes, and smooth muscle cells.

Certain therapeutic agents, however, can react with the bioerodible magnesium alloy to accelerate the erosion of the bioerodible magnesium alloy and/or degrade the therapeutic agent. Accordingly, the therapeutic agent can be segregated from the bioerodible magnesium alloy. In some embodiments, the therapeutic agent is segregated from the bioerodible magnesium alloy with an essentially non-porous and conformal coating. For example, FIGS. 5B-5D depicts stent strut cross-sections having a conformal coating 82 disposed between the therapeutic agent 84 and the bioerodible magnesium alloy of the strut 22.

Therapeutic agents can be combined with a polymer to control the release rate of the drug. Some polymers, however, have been found to irritate the contacted tissues. In addition, some biodegradable polymers generate acidic byproducts and degradation products that elicit an inflammatory response. Moreover, some polymers delay endothelialization of the sent. For example, SIBS delays endothelialization compared to a bare metal stainless steel stent. The stent, accordingly, can be essentially polymer-free (allowing for the presence of any small amounts of polymeric materials that may have been introduced incidentally during the manufacturing process such that someone of ordinary skill in the art would nevertheless consider the coating to be free of any polymeric material). In some embodiments, the rate of drug release can be determined by pore sizes of the coated pores and the rate of diffusion through a porous structure. For example, FIG. 5B depicts a structure that would release the therapeutic agent 84 based on the sizes of the pore openings.

A barrier layer can also be used for controlling the release of the therapeutic agent. FIGS. 5C and 5D depict structures having a barrier coating 86 disposed over therapeutic agent 84. The therapeutic agent may be distributed in a number of ways, including as a continuous layer or discontinuous layer (e.g., the therapeutic agent may be a patterned layer, in pores, or distributed as islands or particles). The barrier layer can be a porous inorganic layer deposited by atomic layer deposition. When the barrier layer is deposited over the therapeutic agent, the deposition temperature may be selected to avoid or reduce heat degradation of the therapeutic agent. For example, a deposition temperature of less than 125 °C may be useful for preserving the therapeutic agent during the deposition process. Deposition temperatures as low as 50 °C may be used for barrier layers such as aluminum oxide.

Various properties of barrier layer 86 will affect the release rate of the therapeutic agent, such as the porosity, thickness, and/or the degradability of coating 86. The porosity and/or degradability of coating 86 may depend upon its composition. For example, an inorganic coating formed of aluminum oxide, zinc oxide, or silicon oxide may be more porous and/or degrade more rapidly (e.g., within days or weeks after immersion in an aqueous solution or implantation in a patient's body) than a titanium oxide coating of the same thickness. In some cases, the inorganic coating degrades completely within 4 weeks after implantation of the medical device in a patient's body.

FIG. 5C depicts a porous stent strut 22 having a conformal non-porous coating 82, one or more therapeutic agents 84 within the pores, and a barrier layer 86 disposed over coating 82 and the therapeutic agent 84. In addition to controlling the release of the therapeutic agent, the barrier layer can smooth the outer surface of porous stent struts.

FIG. 5D depicts a non-porous strut having a conformal non-porous coating 82, a therapeutic agent 84, and a barrier layer 86. A non-porous coating 82 can protect the bioerodible magnesium from the therapeutic agent and also delay the bioerosion of the bioerodible magnesium alloy. The non-porous coating 82 may be titanium oxide deposited by atomic layer deposition. In some embodiments, the non-porous coating 82 can have a thickness of about 2 nm. A barrier layer 86 can overlie the therapeutic agent 84 and control the release of the therapeutic agent. Part of the barrier layer 86 can be deposited prior to depositing the therapeutic agent 84 as a primer layer to increase the adhesion of the therapeutic agent to the strut. The barrier layer 86 may be aluminum oxide deposited by atomic layer deposition. The barrier layer 86 can have a thickness of about 15 nm. The therapeutic agent can be applied to a coated stent strut in a liquid solution and, upon drying, the therapeutic agent can become distributed into therapeutic agent deposits 84. In an alternate embodiment, instead of deposits 84, the therapeutic agent may be provided as a continuous layer.

The stent can also include one or more imaging markers. Imaging markers can assist a physician with the placement of the stent. Imaging markers can be radiopaque marks to permit X-ray visualization of the stent. In some embodiments, the stent can include gold nanoparticles in a carrier. The carrier can be organic or inorganic. For example, the carrier could be a polymer (e.g., poly(lactic-co-glycolic acid)) or a fatty acid (e.g., a triglyceride). In some embodiments, the gold nanoparticles can be positioned within pores. The nanoparticles can also include an inorganic coating deposited using atomic layer deposition (e.g., titanium oxide). The imaging markers can be placed at select locations on the stent.

The coating and/or barrier layer may be capable of undergoing a photocatalytic effect such that the coating becomes superhydrophilic. For example, titanium oxide coatings can be made superhydrophilic and/or hydrophobic using the technique described in U.S. Patent Application Publication No. 2008/0004691 titled "Medical Devices With Selective Coating" (by Weber et al., for Application Serial No. 11/763,770). For example, after a titanium oxide coating is applied over a medical device, the medical device can be placed in a dark environment to cause the titanium oxide coating to become hydrophobic, followed by exposure of the coating (or selected portions of the coating) to UV light to cause the coating (or selected portions) to become superhydrophilic (i.e., such that a water droplet on the coating would have a contact angle of less than 5°). Superhydrophilic coatings can be useful for carrying therapeutic agents, providing a more biocompatible surface for the stent, and/or promoting adherence of endothelial cells to the stent. By selectively making some portions of the coating more hydrophilic or hydrophobic relative to other portions, it may be possible to selectively apply other materials, such as drugs or other coating materials, onto the stent or into pores of the stent based on the hydrophilicity or hydrophobicity of these other materials. For example, the abluminal side of conformal coating 82 can be made superhydrophilic by UV light exposure through a fiber optic line inserted within the lumen of stent 20, or the luminal side of the conformal coating 82 can be made superhydrophilic by exposing the exterior of stent 20 to UV light.

Stent 20 can be configured for vascular, e.g., coronary and peripheral vasculature or non-vascular lumens. For example, it can be configured for use in the esophagus or the prostate. Other lumens include biliary lumens, hepatic lumens, pancreatic lumens, and urethral lumens.

Stent 20 can be of a desired shape and size (e.g., coronary stents, aortic stents, peripheral vascular stents, gastrointestinal stents, urology stents, tracheal/bronchial stents, and neurology stents). Depending on the application, the stent can have a diameter of between, e.g., about 1 mm to about 46 mm. In certain embodiments, a coronary stent can have an expanded diameter of from about 2 mm to about 6 mm. In some embodiments, a peripheral stent can have an expanded diameter of from about 4 mm to about 24 mm. In certain embodiments, a gastrointestinal and/or urology stent can have an expanded diameter of from about 6 mm to about 30 mm. In some embodiments, a neurology stent can have an expanded diameter of from about 1 mm to about 12 mm. An abdominal aortic aneurysm (AAA) stent and a thoracic aortic aneurysm (TAA) stent can have a diameter from about 20 mm to about 46 mm. The stent can be balloon-expandable, self-expandable, or a combination of both (e.g., see U.S. Patent No. 6,290,721).

Non-limiting examples of medical devices that can be used with stent 20 include stent grafts, heart valves, artificial hearts, and other devices that can be used in connection with stent structure. Such medical devices are implanted or otherwise used in body structures, cavities, or lumens such as the vasculature, gastrointestinal tract, abdomen, peritoneum, airways, esophagus, trachea, colon, rectum, biliary tract, urinary tract, prostate, brain, spine, lung, liver, heart, skeletal muscle, kidney, bladder, intestines, stomach, pancreas, ovary, uterus, cartilage, eye, bone, joints, and the like.

Still further embodiments are within the scope of the following claims.

## Claims

1. A bioerodible stent comprising:
a bioerodible magnesium alloy comprising:
between 7 and 8 weight percent aluminum,
between 0.4 and 0.8 weight percent zinc,
between 0.05 and 0.8 weight percent manganese,
optionally between 0.1 and 1.5 weight percent of a first rare earth metal, and, optionally, in case a first rare earth metal is present,
between 0.1 and 1.5 weight percent of a second rare earth metal,
optionally less than 50 ppm Fe,
with the balance of the alloy being magnesium;
wherein the alloy comprises less than 5 weight percent, in sum, of elements other than magnesium, aluminium, zinc and manganese and; and
wherein the stent comprises a coating that consists of a titanium oxide.
oxide and can optionally contain an organic compound.

2. The stent of claim 1, wherein the first rare earth metal is selected from the group consisting of yttrium, neodymium, lanthanum, and cerium.

3. The stent of claim 1, wherein the bioerodible magnesium alloy comprises less than 3 weight percent of elements other than magnesium, aluminum, zinc, manganese, and rare earth metals.

4. The stent of claim 1, wherein the coating have a maximum thickness of 20 nm.

5. The stent of claim 4, further comprising a therapeutic agent deposited over the coating.

6. The stent of claim 4, wherein the stent further comprises a coating of aluminum oxide that is obtainable by atomic layer deposition over the therapeutic agent and the titanium oxide.

7. The stent of claim 4, wherein the coating comprises an organic compound deposited by molecular layer deposition.

8. The stent of claim 1, wherein the stent is polymer-free.

## Patentansprüche

1. Ein biologisch abbaubarer Stent, umfassend:
eine biologisch abbaubare Magnesiumlegierung, umfassend:
zwischen 7 und 8 Gewichtsprozent Aluminium,
zwischen 0,4 und 0,8 Gewichtsprozent Zink,
zwischen 0,05 und 0,8 Gewichtsprozent Mangan,
wahlweise zwischen 0,1 und 1,5 Gewichtsprozent eines ersten Seltenerdenmetalls,
und, wahlweise, falls ein erstes Seltenerdenmetall vorhanden ist, zwischen 0,1 und 1,5 Gewichtsprozent eines zweiten Seltenerdenmetalls,
wahlweise weniger als 50 ppm Fe,
wobei der Rest der Legierung Magnesium ist;
wobei die Legierung in Summe weniger als 5 Gewichtsprozent an von Magnesium, Aluminium, Zink und Mangan verschiedenen Elementen umfasst; und
wobei der Stent eine Beschichtung umfasst, die aus einem Titanoxid besteht und wahlweise eine organische Verbindung enthalten kann.

2. Der Stent nach Anspruch 1, wobei das erste Seltenerdenmetall gewählt ist aus der Gruppe bestehend aus Yttrium, Neodym, Lanthan und Cer.

3. Der Stent nach Anspruch 1, wobei die biologisch abbaubare Magnesiumlegierung weniger als 3 Gewichtsprozent an von Magnesium, Aluminium, Zink und Mangan und Seltenerdenmetallen verschiedenen Elementen umfasst.

4. Der Stent nach Anspruch 1, wobei die Beschichtung eine maximale Dicke von 20 nm aufweist.

5. Der Stent nach Anspruch 4, weiter umfassend ein therapeutisches Mittel, welches über der Beschichtung abgeschieden ist.

6. Der Stent nach Anspruch 4, wobei der Stent weiter eine Beschichtung aus Aluminiumoxid umfasst, welche erhältlich ist durch Atomlagenabscheidung über dem therapeutischen Mittel und dem Titanoxid.

7. Der Stent nach Anspruch 4, wobei die Beschichtung eine organische Verbindung umfasst, die mittels Molekularschichtabscheidung abgeschieden ist.

8. Stent nach Anspruch 1, wobei der Stent polymerfrei ist.

## Revendications

1. Stent bioérodable comprenant :
un alliage de magnésium bioérodable comprenant :
entre 7 et 8 pour cent en poids d'aluminium,
entre 0,4 et 0,8 pour cent en poids de zinc,
entre 0,05 et 0,8 pour cent en poids de manganèse,
éventuellement entre 0,1 et 1,5 pour cent en poids d'une première terre rare,
et, éventuellement, dans le cas où une première terre rare est présente,
entre 0,1 et 1,5 pour cent en poids d'une deuxième terre rare,
éventuellement moins de 50 ppm de Fe,
le reste de l'alliage étant du magnésium ;
l'alliage comprenant moins de 5 pour cent en poids, au total, d'autres éléments que le magnésium, l'aluminium, le zinc et le manganèse ; et
le stent comprenant un revêtement qui est composé d'oxyde de titane et peut éventuellement contenir un composé organique.

2. Stent de la revendication 1, dans lequel la première terre rare est choisie dans le groupe constitué par l'yttrium, le néodyme, le lanthane, et le cérium.

3. Stent de la revendication 1, dans lequel l'alliage de magnésium bioérodable comprend moins de 3 pour cent en poids d'autres éléments que le magnésium, l'aluminium, le zinc, le manganèse, et les terres rares.

4. Stent de la revendication 1, dans lequel le revêtement a une épaisseur maximale de 20 nm.

5. Stent de la revendication 4, comprenant en outre un agent thérapeutique déposé sur le revêtement.

6. Stent de la revendication 4, le stent comprenant en outre un revêtement d'oxyde d'aluminium qui peut être obtenu par dépôt de couches atomiques sur l'agent thérapeutique et l'oxyde de titane.

7. Stent de la revendication 4, dans lequel le revêtement comprend un composé organique déposé par dépôt de couches atomiques.

8. Stent de la revendication 1, le stent étant sans polymère.
